# FASCICULE DE BREVET EUROPEEN

(11) **EP 1 597 376 B1**
(45) Date de publication et mention de la délivrance du brevet: **19.12.2007**
(21) Numéro de dépôt: 04715355.6
(22) Date de dépôt: 27.02.2004
(51) Int. Cl.: C12N 15/86

(54) **PROMOTEUR DE L'ARN POLYMERASE I DE POULET ET SON UTILISATION**
HÜHNER-RNA-POLYMERASE-I-PROMOTOR UND DEREN VERWENDUNG
CHICKEN RNA POLYMERASE I PROMOTER AND THE USE THEREOF

(30) Priorité: 28.02.2003 CA 2421086
(43) Date de publication de la demande: 23.11.2005
(73) Titulaire: INSTITUT PASTEUR, 75015 Paris (FR); CENTRE NATIONAL DE LA RECHERCHE SCIENTIFIQUE (CNRS), 75016 Paris (FR); UNIVERSITE PARIS 7 - Denis DIDEROT, F-75005 Paris (FR)
(72) Inventeur: NAFFAKH, Nadia, F-92240 Malakoff (FR); MASSIN, Pascale, F-22000 Saint Brieuc (FR); VAN DER WERF, Sylvie, F-91190 Gif sur Yvette (FR)
(74) Mandataire: Ahner, Francis
(86) Numéro de dépôt international: PCT/FR2004/000460
(87) Numéro de publication internationale: WO 2004/078912

(56) Documents cités:
- GUILLEMOT F. ET AL: "A MOLECULAR MAP OF THE CHICKEN MAJOR HISTOCOMPATIBILITY COMPLEX: THE CLASS II BETA GENES ARE CLOSELY LINKED TO THE CLASS I GENES AND THE NUCLEOLAR ORGANIZER" EMBO JOURNAL, OXFORD UNIVERSITY PRESS, SURREY, GB, vol. 7, no. 9, 1988, pages 2775-2785, XP002074405 ISSN: 0261-4189 cité dans la demande
- NEUMANN G. ET AL: "Generation of influenza A viruses entirely from cloned cDNAs" PROCEEDINGS OF THE NATIONAL ACADEMY OF SCIENCES OF USA, NATIONAL ACADEMY OF SCIENCE. WASHINGTON, US, vol. 96, août 1999 (1999-08), pages 9345-9350, XP002150093 ISSN: 0027-8424 cité dans la demande
- SCHMID M. ET AL: "First report on chicken genes and chromosomes 2000" 2000, CYTOGENETICS AND CELL GENETICS, VOL. 90, NR. 3-4, PAGE(S) 169-218 , XP009035166 ISSN: 0301-0171 cité dans la demande le document en entier
- NEUMANN G. ET AL: "Reverse genetics of influenza virus" 1 septembre 2001 (2001-09-01), VIROLOGY, VOL. 287, NR. 2, PAGE(S) 243-250 , XP002291927 ISSN: 0042-6822 cité dans la demande le document en entier

## Description

La présente invention concerne un nouveau polynucléotide possédant une activité promotrice de transcription, des vecteurs contenant ce polynucléotide et leur utilisation pour la transcription de séquences d'intérêt, telle que pour la production de virus ARN non coiffé. La présente invention concerne également les cellules hôtes, préférablement d'origine aviaire, contenant un polynucléotide ou un vecteur de l'invention.

La prévention de la grippe repose essentiellement sur la vaccination, qui est fortement recommandée. Elle est par exemple prise en charge à 100 % en France par la Caisse Nationale d'Assurance Maladie, chez les sujets à risques : essentiellement les personnes âgées de 65 ans et plus, et les sujets atteints d'affections chroniques (respiratoires, cardiovasculaires, rénales, ou métaboliques). L'immunité post-vaccinale est conférée par la production d'anticorps dirigés contre les glycoprotéines de surface, notamment contre l'hémagglutinine (HA) mais aussi contre la neuraminidase (NA).

### I- Les méthodes actuelles : production sur oeuf de poule embryonné, et inactivation

Les vaccins antigrippaux sont composés de trois souches de virus différentes, l'une de type A(H3N2), une autre de type A(H1N1) et la troisième de type B. Le choix des souches vaccinales est reconsidéré chaque année en fonction des données de la surveillance des variants en circulation, et fait l'objet d'une recommandation émise par l'OMS vers la mi-février pour l'hémisphère nord. Les vaccins couramment utilisés depuis une trentaine d'années sont composés de virus multipliés sur oeuf de poule embryonné et inactivés (Manuguerra, 2001, Repères sur les infections bronchopulmonaires, pp. 328-342. Edited by P. Léophonte & Y. Mouton: PIL).

Pour les virus de type A, des virus réassortants à haut pouvoir de multiplication sur oeuf embryonné et possédant les HA et les NA des variants indiqués par l'OMS sont préparés et fournis aux producteurs industriels de vaccins. Il faut compter un à deux oeufs embryonnés pour fabriquer une dose de vaccin trivalent. La purification est réalisée de manière différente selon la société de fabrication, mais en suivant le même principe : le liquide allantoïque est prélevé et les virus sont inactivés par traitement au formaldéhyde ou à la β-propiolactone, avant d'être purifiés par ultracentrifugation. Ils peuvent éventuellement être fragmentés à l'aide de solvants de lipides et/ou de détergents tels que le Tween 80. Les virus entiers ou fragmentés sont ensuite ajustés à la dose habituelle de 15 µg de HA par dose de vaccin, pour chacune des souches. Une étape de purification en présence de détergent dialysable permet de produire un vaccin sous-unitaire, qui ne contient plus essentiellement que les glycoprotéines d'enveloppe (HA et NA) du virus. Du fait de la plus forte réactogénicité du vaccin à base de virus entier, ce sont surtout les vaccins fragmentés et sous-unitaires qui sont désormais commercialisés. Le délai nécessaire à la production et au contrôle de la qualité du vaccin avant sa mise sur le marché en conformité avec les normes européennes est de l'ordre de 6 mois.

Composés de virus inactivés, les vaccins antigrippaux sont généralement très bien tolérés, et ne provoquent que de très faibles effets indésirables : réactions locales au point d'injection, réactions fébriles et céphalées dans les deux jours suivant l'injection. Les contre-indications de la vaccination antigrippale se limitent aux allergies aux protéines de l'oeuf, principalement l'ovalbumine, et aux allergies à des résidus de fabrication ou au mercuro-thiolate. En l'absence d'information sur l'effet qu'elle peut avoir sur le développement foetal, la vaccination antigrippale est déconseillée chez la femme enceinte au premier trimestre de la grossesse.

L'efficacité avec laquelle les vaccins composés de virus inactivés protègent contre une infection grippale subséquente est difficile à évaluer, car elle peut varier énormément d'une saison à l'autre, en particulier en fonction du degré de parenté antigénique entre les virus circulants et ceux inclus dans le vaccin, et elle dépend également du statut immunitaire du vacciné. En France, l'amélioration de la couverture vaccinale entre 1979 et 1999 s'est accompagnée d'une diminution de la mortalité liée à la grippe (environ 20 000 morts par an en 1979, 2 500 morts par an depuis 1985), ce qui suggère, sans pour autant le démontrer, un impact positif de la politique vaccinale. Par ailleurs, la compilation de nombreuses études a permis d'estimer que la vaccination chez les personnes âgées permet de diminuer de l'ordre de 50 % le nombre de pneumonies virales et d'hospitalisations, et de l'ordre de 70 % le nombre de cas de grippe mortelle (Gross et al., 1995, Annals of Internal Médecine, 123, 518-527).

### II- Les perspectives d'évolution des vaccins antigrippaux

Plusieurs voies de recherche sont actuellement explorées dans le but 1) d'améliorer l'intensité, la qualité, et la durée des réponses induites par le vaccin antigrippal, et d'élargir leur spécificité ; et 2) de permettre une réponse rapide en cas d'émergence d'un virus de sous-type nouveau potentiellement pandémique. Les travaux concernant la production de vaccins vivants, d'une part, et la production de virus vaccinaux sur culture de cellules, d'autre part, sont résumés ci-dessous. Parmi les autres voies de recherche explorées, on peut citer également l'utilisation d'adjuvants, de vaccins sous-unitaires recombinants ou de vaccins polynucléotidiques.

### - Production de vaccins vivants

Un vaccin antigrippal vivant est susceptible d'induire une immunité plus large et plus durable, en particulier du fait de la stimulation de l'immunité à médiation cellulaire, et d'être mieux accepté par la population générale, parce qu'administré par voie nasale et non par voie parentérale. Deux types de vaccins vivants peuvent aujourd'hui être envisagés : les vaccins vivants atténués et les vaccins vivants recombinants.

### Vaccins vivants atténués

Les souches atténuées sont obtenues par réassortiment des souches sauvages circulantes avec une souche donneuse atténuée par adaptation au froid, dont le génotype est bien caractérisé, sans toutefois que les relations entre les mutations observées et le phénotype (d'atténuation, d'adaptation au froid, de thermo sensibilité) ne soient parfaitement établies (Keitel et al., 1998, Textbook of Influenza, pp. 373-390. Edited by K. G. Nicholson, R. G. Webster & A. J. Hay. Oxford, England: Blackwell Science, Ltd.).

Le procédé de production des vaccins atténués sur oeuf de poule embryonné est analogue à celui décrit ci-dessus, l'étape d'inactivation du virus étant bien entendu supprimée. Les résultats d'essais cliniques menés récemment par des laboratoires américains indiquent que des vaccins trivalents composés de souches atténuées administrés par voie intranasale sont inoffensifs chez l'adulte, les personnes âgées et les enfants, avec comme seuls effets secondaires l'irritation de la gorge et un écoulement nasal pendant les deux jours suivant la vaccination. Ils pourraient avoir une efficacité protectrice supérieure à celle des vaccins inactivés, du fait en particulier de l'induction d'une réponse mucosale en anticorps de type IgA, mais leur potentiel de transmission et de réversion reste à documenter. La vaccination à l'aide de virus vivants atténués est pratiquée à large échelle en Russie depuis plusieurs dizaines d'années, mais il est difficile d'en tirer des résultats globaux convaincants à cause d'une très grande dispersion des conditions employées.

### Vaccins vivants recombinants

La mise au point récente de systèmes de génétique inverse pour les virus grippaux (Neumann et al., 2001, Virology 287, 243-50) permet d'envisager la production de virus recombinants dans le génome desquels des modifications génétiques auraient été introduites spécifiquement afin de leur conférer des propriétés d'atténuation et d'immunogénicité adéquates. Cette approche présenterait une sécurité accrue (du fait de l'absence d'agents contaminants du type de ceux pouvant être présents dans les prélèvements à l'origine des souches virales, et de la possibilité de minimiser et contrôler plus aisément les risques de réversion du virus vaccinal) et permettrait de répondre de manière plus spécifique à des situations épidémiques variées. Les systèmes de génétique inverse existant à ce jour reposent sur l'utilisation du système de transcription de l'ARN polymérase I humaine. Du fait de la spécificité d'espèce étroite de l'ARN polymérase I, leur utilisation est restreinte à des cellules d'origine humaine ou de primates. Ils pourraient s'appliquer à la production de virus vaccinaux recombinants sur culture de cellules, mais non sur oeuf embryonné de poule.

### - Production de virus vaccinaux sur culture de cellules

Les principaux avantages des oeufs embryonnés de poule comme support de la production de virus grippaux vaccinaux sont : i) un rendement de virus très élevé ; et ii) le moindre risque de contamination par des agents pathogènes pour l'homme, qui est tout particulièrement à prendre en compte dans la perspective de la production de vaccins non inactivés. La possibilité de produire des virus vaccinaux sur des cellules cultivées en milieu dépourvu de sérum est aussi explorée. Les industriels Baxter et Solvay ont récemment fait homologuer des banques de cellules dérivées des lignées Vero et MDCK, respectivement, pour la production de virus vaccinaux. Un vaccin produit sur MDCK et inactivé a obtenu l'autorisation de mise sur le marché en 2001, mais n'est pas encore commercialisé.

Le futur verra probablement co-exister sur le marché plusieurs types de vaccins anti-grippaux, inactivés ou vivants, produits sur oeuf embryonné de poule ou sur cellules en culture, parmi lesquels certains se révèleront peut-être particulièrement indiqués pour la vaccination d'une certaine catégorie de sujets, et moins indiqués pour une autre. En cas de pandémie, tous les moyens de production disponibles, sur oeufs embryonnés et sur cellules en culture, devront être mobilisés.

La présente invention répond à ces besoins et à d'autres besoins comme cela sera apparent à une personne versée dans le domaine à la lecture de la présente description de l'invention.

La présente invention concerne un polynucléotide, des ADN recombinants contenant ce polynucléotide et leur utilisation, préférablement pour la production de virus à ARN non coiffé pour fin de vaccination.

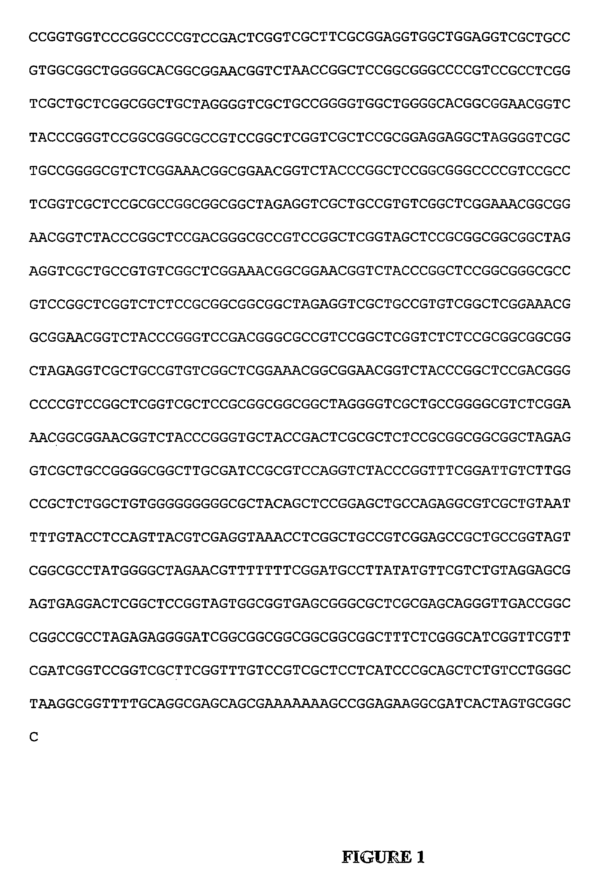

Plus spécifiquement, la présente invention a pour objet un polynucléotide isolé ou purifié qui consiste en la séquence SEQ ID NO: 1, présentée à la figure 1, ou un fragment de celui-ci, et qui possède une activité promotrice de transcription.

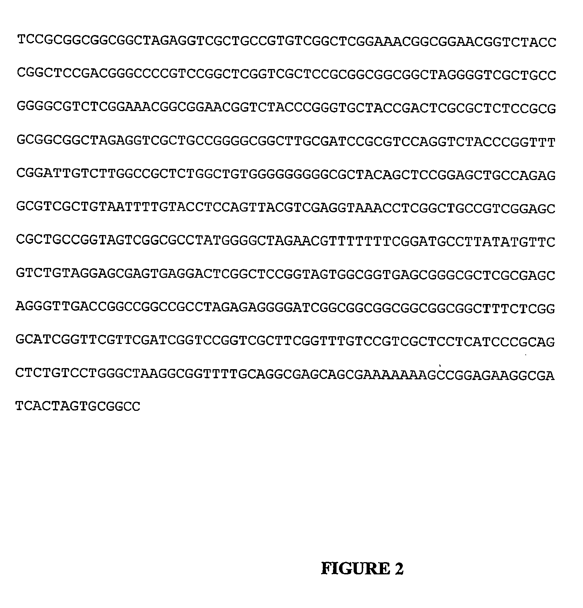

La présente invention concerne également un polynucléotide isolé ou purifié consistant en la séquence SEQ ID NO: 2, présentée à la figure 2, ou un fragment de celui-ci, et possédant une activité promotrice de transcription.

L'invention concerne en outre un ADN recombinant comprenant un des polynucléotides de l'invention ou un fragment de celui-ci ; un vecteur comprenant un des polynucléotides de l'invention ou un ADN recombinant ou un fragment de ceux-ci ; une cellule hôte et un oeuf embryonné d'origine aviaire contenant ledit vecteur.

L'invention porte également sur l'utilisation d'au moins un des éléments suivants pour la production d'une séquence d'intérêt telle que par exemple un virus à ARN non coiffé recombinant :
- un polynucléotide selon l'invention ;
- un ADN recombinant selon l'invention ;
- un vecteur selon l'invention ;
- une cellule hôte selon l'invention ; et
- un oeuf embryonné d'origine aviaire selon l'invention.

Dans une mise en oeuvre particulière, l'invention concerne une méthode de production de virus non coiffés recombinants par génétique inverse, caractérisée en ce qu'elle comprend les étapes suivantes :
a) introduction dans une cellule ou dans un oeuf embryonné d'origine aviaire d'un ou de plusieurs vecteurs, ledit ou lesdits vecteurs comprenant un ADN recombinant selon l'invention et les ADN exprimant les protéines nécessaires à la transcription/réplication de l'ARN viral ;
b) application à la cellule ou à l'oeuf obtenu en a) de conditions permettant l'expression de virus recombinants ; et
c) récupération des virus recombinants obtenus en b).

Dans une mise en oeuvre préférée, la présente invention propose une méthode de production de virus grippaux recombinants par génétique inverse, caractérisée en ce qu'elle comprend les étapes suivantes :
a) introduction dans une cellule aviaire ou dans un oeuf embryonné de poule d'au moins un vecteur comprenant un ADN recombinant selon un mode préféré de l'invention et de vecteurs exprimant les protéines PA, PB1, PB2 et NP d'un virus grippal ;
b) application à la cellule ou à l'oeuf obtenu en a) de conditions permettant l'expression de virus grippaux recombinants ; et
c) récupération des virus grippaux recombinants obtenus en b).

Le clonage du promoteur de l'ARN polymérase I de poulet permet avantageusement de mettre au point un système de génétique inverse adapté à la production de virus vaccinaux recombinants sur oeuf embryonné de poule, qui pourrait s'avérer particulièrement adaptée à la production de vaccins vivants.

### BRÈVE DESCRIPTION DES FIGURES

La Figure 1 montre une séquence (SEQ ID NO: 1) correspondant au fragment AgeI-SacI de l'ADN génomique de poulet contenant le promoteur de l'ARN polymérase I de poulet.
La Figure 2 montre un second fragment d'ADN génomique de poulet (BsaI-SacI) de séquence SEQ ID NO: 2 contenant le promoteur de l'ARN polymérase I de poulet.
La Figure 3 montre le protocole utilisé pour analyser l'efficacité de transcription de la séquence CAT à partir des séquences dérivées du cosmide C13-18.
La Figure 4 montre les résultats de l'analyse itérative de fragments de restriction dérivés du fragment PacI/NotI dérivé du cosmide C13-18.
La Figure 5 montre le résultat de réactions de séquençage et d'extension d'amorce menée en parallèle, qui mettent en évidence, au sein du fragment AgeI/SacI, décrit à la figure 1, le principal site d'initiation de la transcription par l'ARN polymérase I de poulet.
La Figure 6 est un schéma montrant les niveaux de CAT mesurés dans les extraits cytoplasmiques de cellules transfectées avec des plasmides codant pour des pseudo ARN grippaux sous contrôle des promoteurs PolI humain ou de poulet (en ng/10⁶ cellules transfectées et à 24 h post-transfection).

L'originalité de la présente invention porte sur une séquence polynucléotidique qui possède préférablement une activité promotrice de transcription, principalement dans les cellules aviaires. Les inventeurs ont identifié cette séquence promotrice comme étant le promoteur de l'ARN polymérase I de poulet.

L'invention concerne aussi l'implication de ce polynucléotide dans la production de séquences d'intérêts, telles que par exemple des virus grippaux recombinants selon le système de génétique inverse.

### 1. Polynucléotide et ADN recombinant

Selon un premier aspect, l'invention vise un polynucléotide isolé ou purifié consistant en la séquence SEQ ID NO: 1 présentée à la figure 1 ou en la séquence SEQ ID NO: 2 présentée à la figure 2, ou un fragment de celles-ci. Ce polynucléotide, ou l'un de ses fragments, possède une activité promotrice de transcription.

Par fragments, on préfère les fragments dont la séquence comprend au moins 10, 12, 15, 18, 20, 25, 30, 35, 40, 45, 50, 60, 75 ou 100 nucléotides consécutifs de la séquence SEQ ID NO: 1 ou SEQ ID NO: 2 et possédant une activité promotrice de transcription.

Bien que le polynucléotide ou le promoteur de l'invention se caractérise préférablement par le fait qu'il possède une activité promotrice de transcription dans les cellules aviaires, ce promoteur possède particulièrement la capacité de promouvoir la transcription dans les cellules de volaille, et encore plus particulièrement dans les cellules de poulet.

Par séquence nucléotidique, acide nucléique, séquence nucléique ou d'acide nucléique, polynucléotide, séquence polynucléotidique, termes qui seront employés indifféremment dans la présente demande, on entend désigner un enchaînement précis de nucléotides, modifiés ou non, permettant de définir un fragment ou une région d'un polynucléotide, comportant ou non des nucléotides non naturels, et pouvant correspondre aussi bien à un ADN double brin ou à un ADN simple brin. Ceci inclut également les molécules d'ADN, les molécules d'ARN, les ADNc, les séquences artificielles et tous les fragments de ceux-ci. Il va de soi que les définitions "dérivé", "variant" et "muté" s'appliquent également aux polynucléotides selon la présente invention. Tout polynucléotide qui a été modifié chimiquement, enzymatiquement ou métaboliquement mais qui a conservé les propriétés du polynucléotide d'origine, c'est-à-dire, l'activité promotrice de transcription dans les cellules aviaires, est inclus dans la portée de la présente invention.

Il doit être compris que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par clonage, amplification et/ou synthèse chimique, leur environnement ayant été au moins partiellement modifié. Il doit être compris qu'un polynucléotide qui est introduit dans un organisme par transformation, manipulation génétique ou par n'importe quelle autre méthode de recombinaison est "isolé" même si il est présent dans ledit organisme.

Les séquences promotrices selon l'invention possèdent préférablement une séquence d'ADN présentant un pourcentage d'identité de plus de 70 % avec l'une ou l'autre des séquences SEQ ID NO: 1 ou SEQ ID NO: 2 décrites aux figures 1 et 2. Plus particulièrement, les séquences promotrices de l'invention présentent un pourcentage d'identité de plus de 80 %, et encore plus préférablement de 90 % avec l'une ou l'autre des séquences SEQ ID NO: 1 et SEQ ID NO: 2 décrites aux figures 1 et 2, ou un fragment de celles-ci. Par « pourcentage d'identité », on entend un pourcentage qui indique le degré d'identité entre deux séquences d'acides nucléiques le long des séquences dans leur entier. Si les séquences considérées sont de taille différente, le pourcentage d'identité est exprimé en fonction de la longueur totale de la plus courte séquence. Pour calculer le pourcentage d'identité, les deux séquences sont superposées de façon à maximiser le nombre de bases identiques en autorisant des intervalles, puis le nombre de bases identiques est divisé par le nombre total de bases de la plus courte séquence.

Un autre aspect de l'invention concerne un ADN recombinant comprenant un polynucléotide tel que défini ci-dessus.

Cet ADN recombinant peut contenir, par exemple la séquence promotrice SEQ ID NO: 1 présentée à la figure 1 ou SEQ ID NO: 2 présentée à la figure 2, ou un dérivé de celles-ci, dans laquelle est inséré un site de clonage, facilitant ainsi l'utilisation de cette séquence comme promoteur «portable ». Selon un mode préféré, l'ADN recombinant de la présente invention contient en outre une séquence à transcrire. Par « séquence à transcrire » ou « séquence d'intérêt », on entend une séquence pouvant servir de matrice pour synthétiser des molécules d'ARN, telle des ARNv. Préférablement, la séquence à transcrire est un ADNc de virus à ARN non coiffé, c'est-à-dire que les extrémités du produit de transcription ne doivent pas contenir de nucléotides supplémentaires. Plus préférablement, la séquence à transcrire est un ADNc de virus à ARN de polarité négative, tel un orthomyxovirus ou un paramyxovirus. A titre d'exemple, l'orthomyxovirus est un Influenza, préférablement de type A, tandis que le paramyxovirus est préférablement choisi parmi le genre des Rubulavirus (préférablement le virus des oreillons ou le virus de la maladie de Newcastle), le genre des Morbilivirus (préférablement le virus de la rougeole), le genre des Pneumovirus (préférablement le virus respiratoire syncitial) ou le genre des Métapneumovirus. Selon un mode préféré de l'invention, l'ADN recombinant est caractérisé en ce qu'il exprime un ARNv d'un orthomyxovirus, tel un virus grippal et préférablement un ARNv choisi parmi les segments d'ARNv 1 à 8 du virus de l'influenza. La « séquence à transcrire » ou la « séquence d'intérêt » peut également servir pour la production d'ARN anti-sens, c'est-à-dire dont la structure assure, par hybridation avec la séquence cible (par exemple un virus à ARN de polarité négative) une inhibition de la réplication ou transcription d'un ARN viral ou une inhibition de l'expression d'un produit protéique correspondant.

La présente invention concerne également des vecteurs contenant des ADN recombinants comme décrits précédemment. Ces vecteurs permettent l'introduction et éventuellement l'expression de la séquence à transcrire dans une cellule hôte. Comme exemple de vecteurs, on peut citer les vecteurs d'expression plasmidiques, les vecteurs viraux, les vecteurs intégratifs et les vecteurs autosomaux. Plus particulièrement, un vecteur selon la présente invention est le plasmide pGEM-ChPolI-C15. Ce plasmide est contenu dans la souche pGEM-ChPolI-C15-E.Coli 1305 déposée selon le traité de Budapest à la Collection Nationale de Cultures de Micro-organismes (CNCM) Institut Pasteur 28, rue du Dr. Roux, 75724 PARIS (France) le 24 Février 2003 sous le numéro I-2976. Le plasmide pGEM-ChPolI-C15 est dérivé du plasmide pGEM52F+ (Promega). Un fragment de restriction EaeI de 524 pb dérivé de la séquence codant la chloramphénicol acétyle transférase bactérienne (CAT) a été clonée au site NotI de pGEM52F+. Un fragment de restriction AgeI-SacI dérivé du cosmide C13-18 a été cloné au niveau du site EcoRI de pGEM5F+, en amont de l'insert CAT. Ce fragment de 1260 pb contient le promoteur minimal de l'ARN polymérase I de poulet de la présente invention.

L'invention concerne aussi la production d'oiseaux transgéniques, exprimant sous contrôle du promoteur PolI des ARNs conférant une résistance contre des infections par des microorganismes pathogènes, en particulier contre les virus grippaux aviaires.

### 2. Cellule et oeuf embryonné d'origine aviaire

Selon un autre aspect, l'invention concerne des cellules transformées un vecteur tel que décrit précédemment. Préférablement, la cellule hôte ainsi transformée est d'origine aviaire. Selon une variante préférée de l'invention, la cellule hôte est une cellule de volaille, et encore plus préférablement une cellule de poulet. Il est entendu qu'au sens de la présente invention les cellules hôtes transformées le sont préférablement de manière stable. Toutefois, il est envisageable de fournir des cellules transformées de manière transitoire.

Selon un aspect connexe, la présente invention concerne également des oeufs embryonnés « transfectés » d'origine aviaire (préférablement de poule). Plus particulièrement, les oeufs embryonnés de la présente invention comprennent un vecteur tel que décrit précédemment.

Les procédés employés pour introduire un vecteur tel que décrit précédemment dans une cellule hôte ou dans un oeuf embryonné selon la présente invention reposent sur les connaissances générales d'une personne du métier en matière de transfection cellulaire et d'incubation d'oeuf, et ne seront donc pas décrits plus en détails.

### 3. Méthodes et procédé d'utilisation

Selon un autre aspect, l'invention concerne la production de séquences d'intérêts telles que par exemple de virus vaccinaux recombinants, et plus particulièrement la production d'un virus à ARN non coiffé recombinant. Dans un aspect supplémentaire, l'invention vise l'utilisation d'au moins un des éléments suivants pour la production d'une séquence d'intérêt, par exemple d'un virus à ARN non coiffé recombinant :
- un polynucléotide selon l'invention ;
- un ADN recombinant selon l'invention ;
- un vecteur selon l'invention ;
- une cellule hôte selon l'invention ; et
- un oeuf embryonné d'origine aviaire selon l'invention.

Dans un aspect connexe, la présente invention propose une méthode de production de virus non coiffés recombinants. Une telle production se base sur le système de production de virus recombinants par génétique inverse tel que décrit précédemment. Plus particulièrement, la méthode selon la présente invention comprend les étapes suivantes :
a) introduction dans une cellule ou dans un oeuf embryonné d'origine aviaire d'un ou de plusieurs vecteurs, ledit ou lesdits vecteurs comprenant un ADN recombinant selon l'invention et les ADN exprimant les protéines nécessaires à la transcription/réplication de l'ARN viral (comme par exemple les protéines PA, PB 1, PB2 et NP d'un virus grippal) ;
b) application à la cellule ou à l'oeuf obtenu en a) de conditions permettant l'expression de virus recombinants ; et
c) récupération des virus recombinants obtenus en b).

On comprendra qu'à l'étape a), tous les ADN (ADN recombinant selon l'invention et les ADN exprimant les protéines nécessaires à la transcription/réplication de l'ARN viral) peuvent être portés par un seul vecteur ou par deux vecteurs ou plus.

Selon une mise en oeuvre préférée, la présente invention propose également une méthode de production de virus grippaux recombinants, laquelle comprend les étapes suivantes :
a) introduction dans une cellule aviaire ou dans un oeuf embryonné de poule d'au moins un vecteur comprenant préférentiellement un ADN recombinant exprimant un ARNv d'un virus grippal et de vecteurs exprimant les protéines PA, PB1, PB2 et NP d'un virus grippal tel l'influenza, préférablement de type A ;
b) application à la cellule ou à l'oeuf obtenu en a) de conditions permettant l'expression de virus grippaux recombinants ; et
c) récupération des virus grippaux recombinants obtenus en b).

On comprendra que l'étape c) des méthodes de l'invention, soit la récupération des virus recombinants, peut avoir lieu directement sur les cellules, après lyse cellulaire, ou bien à partir du surnageant. Dans le cas où des oeufs embryonnés sont utilisés, une personne versée dans le domaine saura les méthodes à utiliser afin de récupérer les virus recombinants ainsi produits.

Les exemples ci-après permettront de mettre en évidence d'autres caractéristiques et avantages de la présente invention.

### EXEMPLES

Les exemples qui suivent servent à illustrer l'étendue de l'utilisation de la présente invention et non à limiter sa portée. Bien que l'on puisse utiliser d'autres méthodes ou produits équivalents à ceux que l'on retrouve ci-dessous pour tester ou réaliser la présente invention, le matériel et les méthodes préférés sont décrits.

Les systèmes de génétique inverse utilisés afin d'obtenir des virus grippaux recombinants à partir d'ADNc clonés reposent tous sur la transfection de plasmides codant pour les ARN viraux (ARNv) sous le contrôle du promoteur de l'ARN polymérase I humaine (Neumann et al., PNAS 96:9345-50, 1999 ; Fodor et al., J. Virol. 73:9679-82, 1999 ; Hoffman et al., PNAS 97:6108-13, 2000). L'utilisation de ce promoteur se justifie par le fait que les extrémités du produit de transcription ne doivent pas contenir de nucléotides supplémentaires (si c'est le cas, les signaux de transcription/réplication localisés au niveau des extrémités 5' et 3' non codantes des ARNv ne sont plus fonctionnels).

L'utilisation du promoteur de l'ARN polymérase I humaine assure l'initiation de la transcription à un site très précis, et donc l'exactitude de l'extrémité 5' des ARNv synthétisés dans les cellules transfectées. Mais ce type de système ne peut être appliqué que sur des cellules d'origine humaine ou simienne, du fait de la très étroite spécificité d'espèce des promoteurs des ARN polymérase I (Heix and Grummt, Curr. Op. Gent. Dev. 5:652-56, 1995).

Or, disposer d'un système de génétique inverse applicable sur des cellules aviaires pourrait ouvrir la voie à la production de vaccins grippaux recombinants sur des oeufs de poule embryonnés, seul support actuellement autorisé pour la production du vaccin grippal aux Etats-Unis. La vaccination constitue à ce jour l'unique moyen de prévention contre la grippe, et elle est fortement recommandée chez certaines catégories de sujets à risque. La fabrication du vaccin repose sur la multiplication de souches vaccinales sur oeufs embryonnés, et nécessite, à chaque réactualisation de la composition vaccinale, de sélectionner des virus réassortants possédant les segments HA et NA représentatifs des souches circulantes, les autres segments dérivant d'une souche donneuse adaptée à la croissance sur oeuf. Il pourrait être avantageux de remplacer ce processus long et aléatoire par la production de virus réassortants par génétique inverse sur oeuf embryonné.

Ainsi, dans la perspective de mettre au point un système de production de virus grippaux recombinants par génétique inverse sur cellules aviaires, les inventeurs ont entrepris de cloner le promoteur de l'ARN polymérase I de poulet. Un clonage par PCR basé sur les homologies entre séquences nucléotidiques des promoteurs PolI clonés à ce jour n'était pas envisageable, car les systèmes de transcription PolI sont extrêmement divergents d'une espèce à l'autre. Ils ont donc procédé à un criblage de tout ou partie du génome de poulet.

### 1) Obtention du cosmide C13-18

La cartographie du génome du poulet indiquait que les gènes codant les ARN ribosomaux (locus NOR) étaient situés sur le chromosome 16, à proximité du locus des gènes du complexe d'histocompatibilité (Schmid et al., Cytogenet. Cell Genet., 90:169-218, 2000). Le laboratoire du Pr. H. Auffray, dont une partie des travaux à l'Institut Gustave Roussy de Villejuif portent sur le complexe majeur d'histocompatibilité, a publié en 1988 des données concernant un cosmide recombinant (C13-18) contenant 30kb d'ADN génomique de poulet, parmi lesquelles les gènes du complexe majeur d'histocompatibilité, mais aussi plusieurs unités de transcription du locus NOR, et par conséquent au moins un exemplaire du promoteur PolI (Guillemot et al., EMBO J., 7:2775-85, 1988). Ce cosmide a été aimablement fourni par R. Zoorob.

### 2) Analyse du cosmide C13-18 par Southern blot

Le cosmide C13-18 a été analysé par Southern blot après digestion par les enzymes de restriction NotI, PacI, et SfiI, isolément ou en combinaison, et en utilisant comme sonde des oligonucléotides choisis sur la base des données de séquençage partiel des ARN ribosomaux de poulet disponibles dans les banques :
oligonucléotide 28S/+/2 : 5'-GAGTCAGCCCTCGACACAAGCTTTTG-3' (SEQ ID NO: 3) ;
oligonucléotide 18S/-/1.5 : 5'-CTACTGGCAGGATCAACCAGGT-3' (SEQ ID NO: 4) ; et
oligonucléotide 18S/-/4 : 5'-TAGAGGAGAACGCGACCTCGAGAC-3' (SEQ ID NO: 5).

Cette analyse a permis de reconstituer une carte de restriction grossière du cosmide C13-18, et de positionner les ARN ribosomaux 18S et 28S par rapport aux divers sites de restriction localisés sur le cosmide. Elle a débouché sur l'identification d'un fragment PacI-NotI de 16 kb environ, correspondant à la majeure partie d'une région intergénique, et par conséquent très susceptible de contenir une copie de la séquence du promoteur PolI.

### 3) Clonage du fragment de restriction PacI-NotI dans un vecteur "rapporteur", et mise en évidence de la présence d'un promoteur transcriptionnel au sein du fragment PacI-NotI

Afin de tester la présence de la séquence du promoteur Po1I au sein du fragment de restriction PacI-NotI, les inventeurs ont cherché à cloner ce dernier en amont d'une séquence rapporteur.

Dans ce but, le cosmide pTCF (Grosveld et al., 1982, Nucleic Acids Res., 10, 6715-32), qui possède un unique site de clonage SalI, a été modifié, par insertion d'une séquence synthétique contenant des sites de clonages multiples (StuI-PacI-NotI-PmeI) au niveau du site SalI.

Le fragment PacI-NotI de 16 kb dérivé de C13-18 a été cloné dans le cosmide pTCF modifié, au niveau des sites PacI et NotI nouvellement introduits. Deux clones de cosmides recombinants pTCF-(PacI-NotI) ont été sélectionnés (n° 2.5 et 2.7). Puis un fragment de restriction EaeI de 500pb, dérivé du gène bactérien de la chloramphénicol acétyle transférase (CAT) a été cloné au niveau du site NotI des cosmides pTCF-(PacI-NotI), dans l'une et l'autre orientation. Quatre clones de cosmides recombinants ont été sélectionnés : n° 2.5.3 et n° 2.7.13 (CAT en orientation +), n° 2.5.4 et n° 2.7.16 (CAT en orientation -).

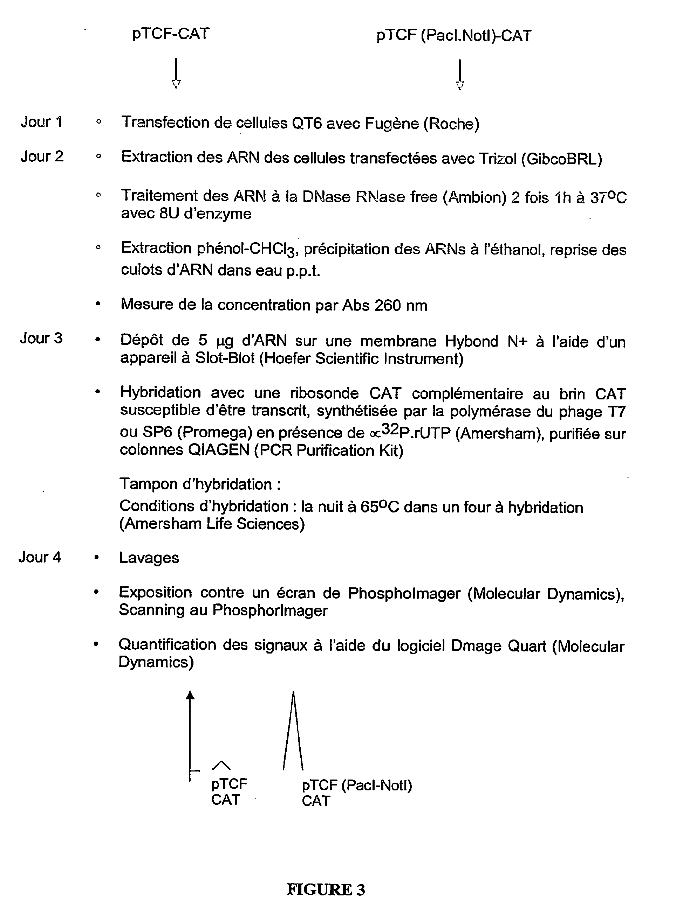

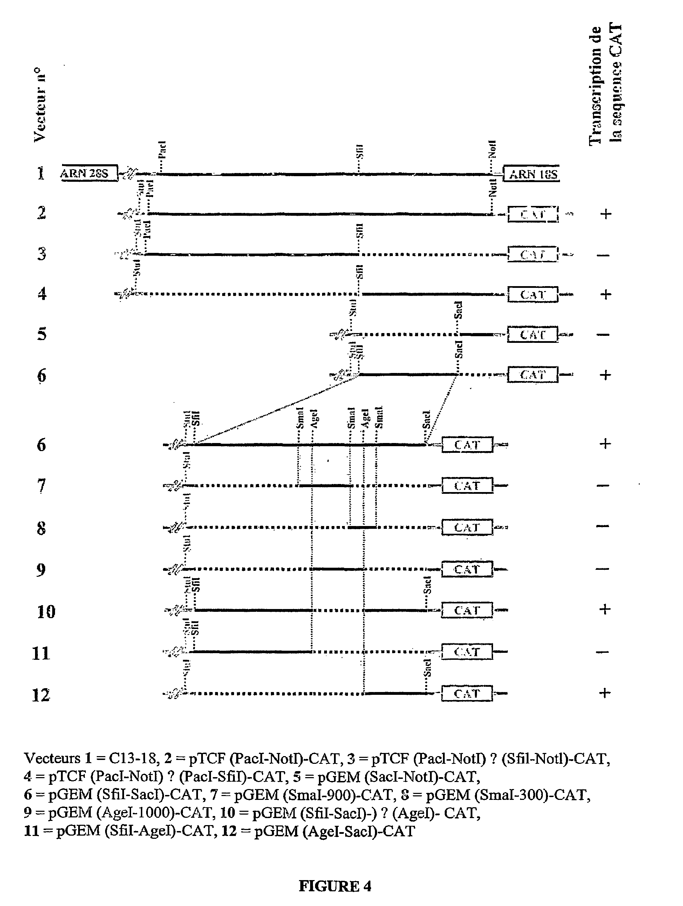

Afin de tester la présence de la séquence du promoteur PolI au sein du fragment de restriction PacI-NotI, des cellules de la lignée QT6 (fibrosarcome de caille) ont été transfectées avec les cosmides pTCF-(PacI-NotI)-CAT(+) et pTCF-(PacI-NotI)-CAT(-). Les ARNs ont été extraits des cellules 24 heures après transfection à l'aide du réactif "TriZol" (Gibco-BRL) et traités à la DNase (RNase-free DNase, Ambion) afin d'éliminer les molécules de cosmide contaminant les préparations d'ARN. Les ARNs ont été déposés sur une membrane de nylon (Hybond N+, Amersham) à raison de 5 µg par point, et hybridés avec une ribosonde marquée au ³²P, correspondant à la séquence CAT(+). Des signaux positifs ont été obtenus avec les ARNs préparés à partir des cellules transfectées avec les cosmides pTCF-(PacI-NotI)-CAT(-) n° 2.5.4 et 2.7.16, suggérant la présence d'un promoteur transcriptionnel dans le fragment de restriction PacI-NotI. Dans ces premières expériences, le rapport signal/bruit de fond était néanmoins assez faible. Il a été amélioré dans les expériences suivantes après optimisation des conditions de traitement des ARNs à la DNase (durant 2 fois 1 heure à 37°C en présence de 8 unités d'enzyme) et des conditions d'hybridation et de lavage des membranes (hybridation durant la nuit à 65°C dans un tampon formamide 50 %, SSC 5X, Denhart 5X, SDS 0,5 % ; 2 lavages de 10 mn à température ambiante dans un tampon SSC 2X, SDS 0,1 %, suivis de 2 lavages de 10 mn à 65°C dans un tampon SSC 0,2X, SDS 0,1 %). La méthodologie, qui a été appliquée ensuite de manière itérative pour examiner la présence d'un promoteur transcriptionnel dans des fragments de restriction dérivés du fragment PacI-NotI, est récapitulée à la figure 3. Les résultats obtenus sont détaillés ci-dessous, et récapitulés à la figure 4.

### 4) Création de délétions partielles au sein du fragment de restriction PacI-NotI cloné dans le vecteur rapporteur, et mise en évidence de la présence d'un promoteur transcriptionnel dans un fragment SfiI-NotI d'environ 6 kb

Afin d'identifier plus précisément la région du fragment PacI-NotI contenant le promoteur, des délétions partielles du fragment PacI-NotI ont été obtenues par digestion des cosmides pTCF-(PacI-NotI)-CAT(-) n° 2.7.16 et pTCF-(PacI-NotI)-CAT(+) n° 2.7.13 avec les combinaisons d'enzymes PacI-SfiI ou SfiI-NotI (le site SfiI étant situé au sein du fragment PacI-NotI), remplissage des extrémités à l'aide de la sous-unité Kleenow de l'ADN PolI d'E. Coli, et religation des molécules obtenues sur elles-mêmes. La présence d'un promoteur transcriptionnel au sein des cosmides délétés a été testée en utilisant la méthodologie décrite ci-dessus (transfection de cellules QT6 et analyse des ARN extraits des cellules transfectées par hybridation avec une sonde CAT). Les résultats obtenus ont suggéré qu'un promoteur transcriptionnel était présent dans les cosmides ayant retenu la portion SfiI-NotI (de 6 kb environ) du fragment PacI-NotI.

### 5) Analyse itérative de fragments de restriction dérivés du fragment SfiI-NotI, et mise en évidence de la présence d'un promoteur transcriptionnel dans un fragment AgeI-SacI de 1200 pb

A cette étape, et afin de poursuivre l'analyse de fragments de restriction dérivés du fragment SfiI-NotI selon la même méthodologie, un nouveau vecteur rapporteur a été construit. En effet, les fragments de restriction analysés avaient désormais une taille suffisamment réduite pour pouvoir être clonés dans un plasmide.

Ce nouveau vecteur rapporteur a été construit à partir du plasmide pGEM-S-zf+ (Promega), dans lequel le fragment EaeI de 500pb dérivé du gène bactérien CAT a été cloné en orientation (+) au niveau du site unique NotI. Seule cette orientation de la séquence CAT dans le vecteur rapporteur a été utilisée, car les expériences précédentes avaient indiqué que le couple séquence rapporteur CAT (+) / ribosonde CAT (-) donnait des résultats plus spécifiques et reproductibles que le couple séquence rapporteur CAT (-)/ribosonde CAT (+).

Les fragments de restriction SfiI-SacI (dans la pratique, un fragment StuI-SacI incluant le fragment SfiI-SacI) et SacI-NotI dérivés du fragment SfiI-NotI ont été sous-clonés au niveau du site EcoRV du vecteur pGEM-CAT(+) (clone n° 9), situé en amont de la séquence rapporteur CAT. Par la méthodologie décrite ci-dessus, la présence d'un promoteur transcriptionnel a été mise en évidence dans le fragment StuI-SacI, cloné dans le plasmide pGEM-(StuI-SacI)-CAT(+) (clone n° 12).

Une carte de restriction du fragment StuI-SacI, reposant sur des digestions du plasmide pGEM-(StuI-SacI)-CAT(+) n° 12 avec les enzymes de restriction SmaI et AgeI, a été établie. Deux fragments de restriction SmaI-SmaI, un fragment de restriction AgeI-AgeI, et le fragment StuI-SacI délété de ce fragment de restriction AgeI-AgeI, ont été sous-clonés dans le vecteur pGEM-CAT(+) n° 9 au niveau du site EcoRV. La présence d'un promoteur transcriptionnel a été mise en évidence dans le fragment StuI-SacI délété du fragment AgeI-AgeI, cloné dans le plasmide appelé par commodité V12-AgeI (clone n° 29).

Le fragment StuI-SacI délété a été découpé en deux fragments StuI-AgeI et AgeI-SacI, obtenus par digestion du plasmide V12-AgeI n° 29 par les enzymes de restriction NcoI + AgeI d'une part (pour le fragment StuI-AgeI), et AgeI + NotI d'autre part (pour le fragment AgeI-SacI). Chacun des deux fragments a été sous-cloné dans le vecteur pGEM-CAT(+) au niveau du site EcoRV. La présence d'un promoteur transcriptionnel a été mise en évidence dans le fragment AgeI-SacI de 1200 pb, cloné dans le plasmide pGEM-(AgcI-SacI)-CAT(+) (clone n° 15).

### 6) Séquençage du fragment AgeI-SacI, et détermination du point d'initiation de la transcription par la technique d'extension d'amorce

Le fragment AgeI-SacI cloné dans le plasmide pGEM-(AgeI-SacI)-CAT(+) n° 15 a été séquencé. La séquence est représentée à la figure 1 (SEQ ID NO: 1).

Les 800 pb distales de cette séquence contiennent 8 répétitions d'une séquence d'environ 90 nucléotides, ce qui est caractéristique des promoteurs PolI : en effet la présence de séquences activatrices répétées en amont du site d'initiation de la transcription a été décrite pour plusieurs promoteurs PolI dérivés d'autres espèces (Paule M., 1998, Promoter Structure of Class I Genes. In Transcription of Ribosomal RNA Genes by Eukaryotic RNA Polymerase I. Edited by M. R. Paule: Springer-Verlag and R.G. Landes Company).

Le site précis de l'initiation de la transcription du promoteur PolI de poulet a donc été recherché dans les 400 pb proximales du fragment AgeI-SacI, par la technique d'extension d'amorce. Un oligonucléotide complémentaire de la séquence CAT transcrite à partir du plasmide pGEM-(AgeI-SacI)-CAT(+) (séquence SEQ ID NO: 6 : 5'-ATGTTCTTTACGATGCGATTGGG-3') a été marqué avec du ³²P à son extrémité 5' à l'aide de la polynucléotide kinase du phage T4 (Biolabs) et utilisé dans deux réactions en parallèle : 1) comme amorce pour l'extension d'un ADN complémentaire des ARN CAT extraits de cellules QT6 transfectées avec le plasmide pGEM-(AgeI-SacI)-CAT(+) n° 15, en présence de transcriptase inverse (SuperScript II, Invitrogen), et 2) comme amorce pour le séquençage du plasmide pGEM-(AgeI-SacI)-CAT(+) n° 15, à l'aide du kit « T7 Sequencing Kit » (Pharmacia Biotech). Un produit de transcription unique a été détecté dans la réaction d'extension d'amorce, confirmant l'existence d'un site très précis d'initiation de la transcription. Ce dernier a été localisé dans une région riche en A et T, en accord avec les données disponibles pour les promoteurs PolI dérivés d'autres espèces, située à environ 200 pb de l'oligonucléotide utilisé comme amorce.

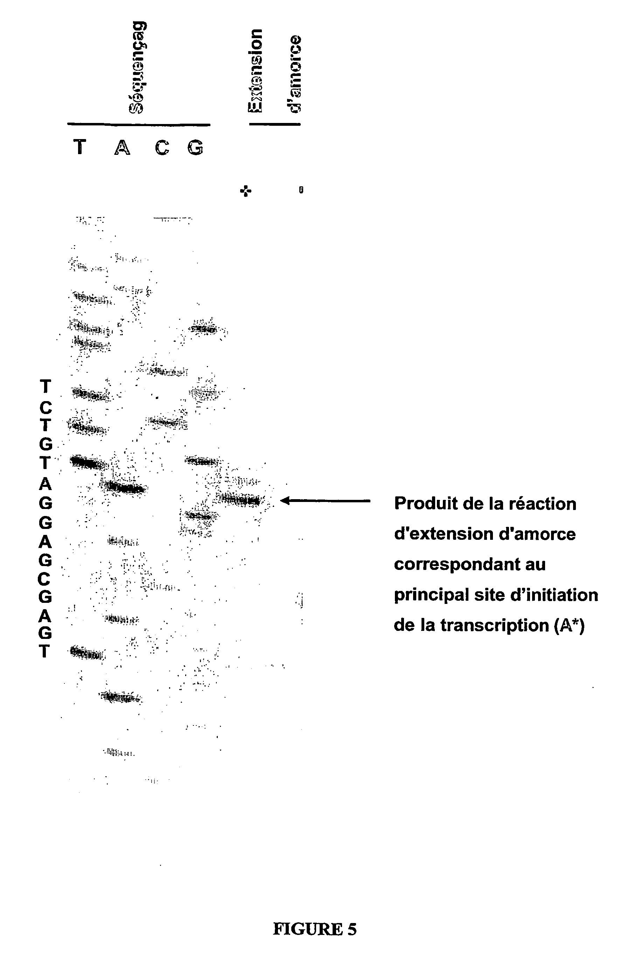

En parallèle, l'analyse de fragments de restriction dérivés du fragment AgeI-SacI a été poursuivie afin de délimiter le promoteur minimal (qui correspond à une région de l'ordre de 250 pb pour les promoteurs PolI d'autres espèces) (voir figure 5). Le fragment AgeI-SacI a été découpé en trois fragments AgeI-BsaI, BsaI-BsaI et BsaI-SacI, obtenus par digestion du plasmide pGEM-(AgeI-SacI)-CAT(+) n° 15 par les enzymes de restriction NcoI + BsaI d'une part (pour les fragment AgeI-BsaI et BsaI-BsaI), et BsaI + NotI d'autre part (pour le fragment BsaI-SacI). Chacun des deux fragments a été sous-cloné dans le vecteur pGEM-CAT(+) au niveau du site EcoRV. Seul le fragment BsaI-SacI d'environ 600 pb (figure 2), cloné dans le plasmide pGEM-(BsaI-SacI)-CAT(+) (clone n° 16), permet la transcription de la séquence rapporteur CAT, ce qui est parfaitement cohérent avec le fait qu'il s'agit du fragment proximal dans lequel a été localisé le site d'initiation de la transcription.

Le nucléotide +1 au site d'initiation de la transcription a été déterminé par la technique d'extension d'amorce, en utilisant un oligonucléotide situé à une cinquantaine de paires de bases en aval de la région d'initiation (voir figure 5). Cet oligonucléotide de séquence SEQ ID NO: 7 (5'-GGCCGGTCAACCCTGCTC-3') a été marqué avec du ³²P à son extrémité 5' à l'aide de la polynucléotide kinase du phage T4 (Biolabs) et utilisé dans deux réactions en parallèle : 1) comme amorce pour l'extension d'un ADN complémentaire des ARN CAT extraits de cellules QT6 transfectées avec le plasmide pGEM-(AgeI-SacI)-CAT(+) n°15, en présence de transcriptase inverse (SuperScript II, Invitrogen), et 2) comme amorce pour le séquençage du plasmide pGEM-(AgeI-SacI)-CAT(+) n° 15, à l'aide du kit « T7 Sequencing Kit » (Pharmacia Biotech). Un produit de transcription majoritaire a été détecté, correspondant à un site d'initiation qui possède un environnement nucléotidique caractéristique (nucléotides soulignés ci-dessous) de la séquence consensus établie d'après les séquence de promoteurs PolI d'autres espèces :

Il faut noter cependant que trois autres types de produits de transcription, minoritaires, ont été détectés dans cette expérience d'extension d'amorce, correspondant à une initiation aux nucléotides -1, +13 et +14 (nucléotides marqués par une étoile ci-dessus).

Des plasmides destinés à exprimer un ARN pseudo-grippal porteur de la séquence du gène rapporteur CAT sous contrôle du promoteur PolI de poulet ont été construits sur le modèle du plasmide pPolI-CAT-Rz conçu par Pleschka et al. à l'aide du promoteur PolI humain (1996, J. Virol., 70:4188-92) : ces plasmides (pPR7-C16-CAT-Rz et pPR7-ΔC16-CAT-Rz) contiennent les séquences codant pour le gène CAT, clonées en anti-sens, et flanquées des séquences 5' et 3' non codantes dérivées du segment génomique NS d'un virus grippal humain de type A ; le positionnement de l'extrémité 5' non codante exactement en aval du nucléotide -1 du promoteur Po1I de poulet, d'une part, et l'insertion de la séquence ribozyme du virus de l'hépatite δ à l'extrémité 3' non codante, d'autre part, sont destinés à assurer l'exactitude des extrémités du produit de transcription. Le plasmide pPR7-C16-CAT-Rz contient les nt-1 à - 425 du promoteur PolI de poulet, alors que le plasmide pPR7-ΔC16-CAT-Rz ne contient que les nt - 1 à - 246 (pour d'autres espèces, la taille du promoteur PolI minimal a été estimée à 250 nt environ).

Les plasmides pPolI-CAT-Rz (promoteur PolI humain), pFR7-C16-CAT-Rz ou pPR7-ΔC16-CAT-Rz (promoteur Po1I aviaire) ont été transfectés dans des cellules aviaires QT6 ou dans les cellules de primate COS-1, simultanément avec des plasmides codant pour les protéines PB1, PB2, PA et NP d'un virus grippal. Si les cellules transfectées expriment des ARN pseudo-grippaux corrects au niveau des extrémités 5' et 3' non codantes, et simultanément les protéines PB1, PB2, PA et NP, les ARN pseudo-grippaux peuvent être transcrits et répliqués, et l'efficacité du processus de transcription/réplication peut être estimée par la mesure du niveau de protéine CAT dans les cellules transfectées. Les niveaux de protéine CAT ont donc été mesurés par ELISA dans les extraits cytoplasmiques des cellules transfectées, préparés à 24 heures post-transfection.

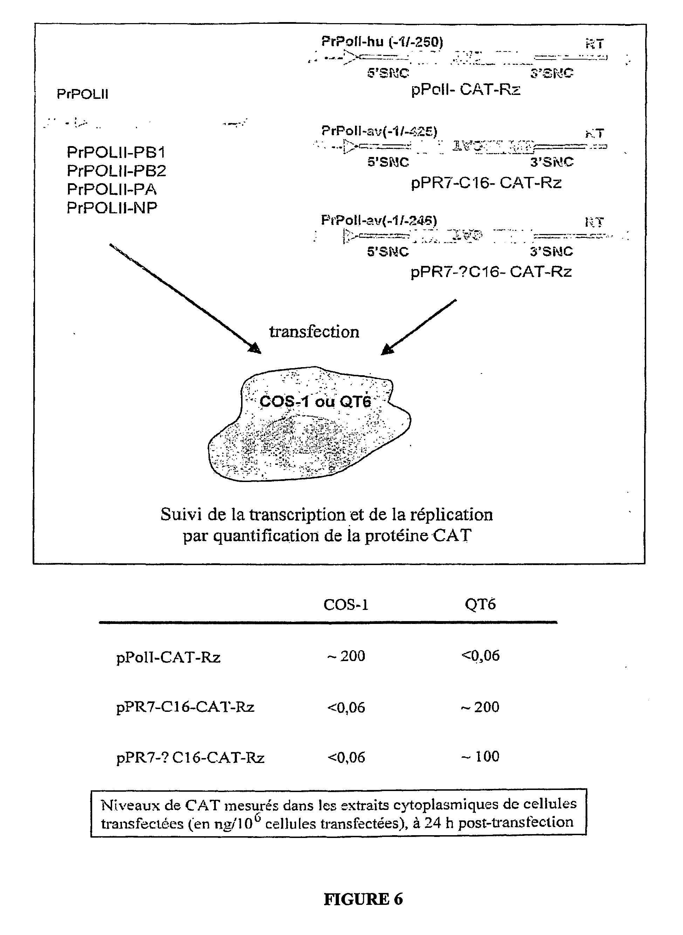

Les niveaux de CAT mesurés en présence du plasmide pPolI-CAT-Rz étaient de l'ordre de 200ng/10⁶ cellules transfectées lorsqu'il s'agissait de cellules COS-1, alors qu'ils étaient très faibles (<0,06ng/10⁶ cellules transfectées) lorsqu'il s'agissait de cellules QT6 (figure 6).

A l'inverse, les niveaux de CAT mesurés en présence des plasmides pPR7-C16-CAT-Rz et pPR7-ΔC16-CAT-Rz étaient très faibles (<0,06ng/10⁶ cellules transfectées) dans les cellules COS-1, mais ils étaient de l'ordre de 200 et 100ng/10⁶ cellules, respectivement, dans les cellules QT6 (figure 6).

Ces résultats établissent que les séquences du promoteur PolI de poulet clonées dans les plasmides pPR7-C16-CAT-Rz et pPR7-ΔC16-CAT-Rz permettent, après transfection dans des cellules d'origine aviaire, la transcription *in vivo* de mini-réplicons comportant les signaux de réplication d'un virus grippal de type A. Ils ouvrent donc la voie au développement de systèmes de génétique inverse permettant d'obtenir des virus grippaux recombinants sur des cellules aviaires en culture, ou directement sur oeufs embryonnés de poule.

## Revendications

1. Polynucléotide isolé ou purifié consistant en la séquence SEQ ID NO: 1 ou un fragment de celui-ci constitué d'au moins 10 nucléotides consécutifs de la séquence SEQ ID NO: 1 et possédant une activité promotrice de transcription.

2. Polynucléotide isolé ou purifié consistant en la séquence SEQ ID NO: 2 ou un fragment de celui-ci constitué d'au moins 10 nucléotides consécutifs de la séquence SEQ ID NO: 2 et possédant une activité promotrice de transcription.

3. Polynucléotide selon la revendication 1 ou 2, **caractérisé en ce qu'**il possède une activité promotrice de transcription dans les cellules aviaires, préférentiellement dans les cellules de volaille, et plus particulièrement dans les cellules de poulet.

4. ADN recombinant comprenant un des polynucléotides définis aux revendications 1 à 3.

5. ADN recombinant selon la revendication 4, **caractérisé en ce qu'**il comprend en outre une séquence à transcrire.

6. ADN recombinant selon la revendication 5, **caractérisé en ce que** la séquence à transcrire est un ADNc de virus à ARN non coiffé.

7. ADN recombinant selon la revendication 6, **caractérisé en ce que** la séquence à transcrire est un ADNc de virus à ARN de polarité négative.

8. ADN recombinant selon la revendication 7, **caractérisé en ce que** le virus à ARN de polarité négative est un orthomyxovirus ou un paramyxovirus.

9. ADN recombinant selon la revendication 8, **caractérisé en ce que** l'orthomyxovirus est un virus de l'influenza, préférablement de type A.

10. ADN recombinant selon la revendication 9, **caractérisé en ce qu'**il exprime un ARNv d'un orthomyxovirus, tel qu'un virus grippal et préférentiellement un ARNv choisi parmi les segments d'ARNv 1 à 8 du virus de l'influenza.

11. ADN recombinant selon la revendication 8, **caractérisé en ce que** le paramyxovirus est choisi parmi le genre des Rubulavirus tel le virus des oreillons et le virus de la maladie de Newcastle, le genre des Morbilivirus tel le virus de la rougeole, le genre Pneumovirus tel le virus respiratoire syncitial et le genre des Métapneumovirus.

12. Vecteur comprenant un polynucléotide selon l'une quelconque des revendications 1 à 3 et/ou un ADN recombinant selon l'une quelconque des revendications 4 à 11.

13. Cellule hôte comprenant un vecteur selon la revendication 12.

14. Cellule hôte selon la revendication 13, **caractérisée en ce qu'**elle est d'origine aviaire.

15. Cellule hôte selon la revendication 14, **caractérisée en ce qu'**elle est une cellule de volaille, et plus particulièrement de poulet.

16. OEuf embryonné d'origine aviaire comprenant un vecteur selon la revendication 12.

17. Utilisation d'au moins un des éléments suivants pour la production d'un virus à ARN de polarité négative recombinant :
- un polynucléotide selon l'une quelconque des revendications 1 à 3 ;
- un ADN recombinant selon l'une quelconque des revendications 4 à 11;
- un vecteur selon la revendication 12 ;
- une cellule hôte selon l'une quelconque des revendications 13 à 15 ; et
- un oeuf embryonné selon la revendication 16.

18. Méthode de production de virus grippaux recombinants par génétique inverse, **caractérisée en ce qu'**elle comprend les étapes suivantes :
a) introduction dans une cellule aviaire ou dans un oeuf embryonné d'origine aviaire d'au moins un vecteur comprenant un ADN recombinant tel que défini à la revendication 9 et de vecteurs exprimant les protéines PA, PB 1, PB2 et NP d'un virus grippal ;
b) application à la cellule ou à l'oeuf obtenu en a) de conditions permettant l'expression de virus grippaux recombinants ; et
c) récupération des virus grippaux recombinants obtenus en b).

19. Méthode selon la revendication 18, **caractérisée en ce que** la cellule est une cellule de volaille, et plus particulièrement de poulet.

20. Méthode selon la revendication 18, **caractérisée en ce que** le virus grippal est l'influenza, préférablement de type A.

21. Méthode de production de virus non coiffés recombinants par génétique inverse, **caractérisée en ce qu'**elle comprend les étapes suivantes :
a) introduction dans une cellule aviaire ou dans un oeuf embryonné d'origine aviaire d'un ou de plusieurs vecteurs, ledit ou lesdits vecteurs comprenant un ADN recombinant selon l'une quelconque des revendications 4 à 11 et les ADN exprimant les protéines nécessaires à la transcription/réplication de l'ARN viral ;
b) application à la cellule ou à l'oeuf obtenu en a) de conditions permettant l'expression de virus recombinants ; et
c) récupération des virus recombinants obtenus en b).

22. Polynucléotide selon la revendication 1 ou 2, **caractérisé en ce qu'**il est contenu dans la souche pGEM-ChPolI-C15-E.Coli 1305 déposée à la CNCM le 24 février 2003 sous le numéro 1-2976.

## Claims

1. An isolated or purified polynucleotide consisting of sequence SEQ ID NO: 1 or a fragment thereof comprised of at least 10 consecutive nucleotides of sequence SEQ ID NO: 1 and that possesses transcription-promoting activity.

2. An isolated or purified polynucleotide consisting of sequence SEQ ID NO: 2 or a fragment thereof comprised of at least 10 consecutive nucleotides of sequence SEQ ID NO: 2 and that possesses transcription-promoting activity.

3. A polynucleotide according to claim 1 or 2, **characterized in that** it possesses a transcription-promoting activity in avian cells, preferentially in poultry cells, and more particularly in chicken cells.

4. A recombinant DNA including one of the polynucleotides defined in claims 1 to 3.

5. A recombinant DNA according to claim 4, **characterized in that** it includes, moreover, a sequence to be transcribed.

6. A recombinant DNA according to claim 5, **characterized in that** the sequence to be transcribed is a cDNA of an uncapped RNA virus.

7. A recombinant DNA according to claim 6, **characterized in that** the sequence to be transcribed is cDNA of a negative-polarity RNA virus.

8. A recombinant DNA according to claim 7, **characterized in that** the negative-polarity RNA virus is an orthomyxovirus or a paramyxovirus.

9. A recombinant DNA according to claim 8, **characterized in that** the orthomyxovirus is an influenza virus, preferably Type A.

10. A recombinant DNA according to claim 9, **characterized in that** it expresses a vRNA of an orthomyxovirus, such as influenza virus and preferentially a vRNA chosen from among influenza virus vRNA segments 1 to 8.

11. A recombinant DNA according to claim 8, **characterized in that** the paramyxovirus is selected from among the genus Rubulavirus, such as the mumps virus and Newcastle's disease virus, the genus Morbillivirus, such as the measles virus, the genus Pneumovirus, such as respiratory syncytial virus, and the genus Metapneumovirus.

12. A vector including a polynucleotide according to any of the claims 1 to 3 and/or a recombinant DNA according to any of the claims 4 to 11.

13. A host cell including a vector according to claim 12.

14. A host cell according to claim 13, **characterized in that** it is of avian origin.

15. A host cell according to claim 14, **characterized in that** it is a poultry cell, and more particularly a chicken cell.

16. An embryonated egg of avian origin including a vector according to claim 12.

17. The use of at least one of the following elements for the production of a negative-polarity recombinant RNA virus:
- a polynucleotide according to any of the claims 1 to 3;
- a recombinant DNA according to any of the claims 4 to 11;
- a vector according to claim 12;
- a host cell according to any of the claims 13 to 15; and
- an embryonated egg according to claim 16.

18. A method for producing recombinant influenza viruses by reverse genetics, **characterized in that** it includes the following steps:
a) introducing into an avian cell or embryonated egg of avian origin of at least one vector including a recombinant DNA as defined in claim 9 and vectors that express influenza virus proteins PA, PB1, PB2, and NP;
b) applying to the cell or egg obtained in a) conditions that make possible the expression of recombinant influenza viruses; and
c) recovering the recombinant influenza viruses obtained in b) .

19. A method according to claim 18, **characterized in that** the cell is a poultry cell, and more particularly a chicken cell.

20. A method according to claim 18, **characterized in that** the influenza virus is preferably Type A.

21. A method of producing non-capped recombinant viruses by reverse genetics, **characterized in that** it includes the following steps:
a) introducing into an avian cell or embryonated egg of avian origin of one or several vectors, the aforementioned vector or vectors comprising a recombinant DNA according to any one of claims 4 to 11, and DNA that express the proteins necessary for transcribing/replicating viral RNA;
b) applying to the cell or egg obtained in a) conditions that make possible the expression of recombinant viruses; and
c) recovering the recombinant viruses obtained in b).

22. A polynucleotide according to claim 1 or 2, **characterized in that** it is contained in the strain pGEM-ChPolI-C15-E.Coli 1305 deposited with the CNCM on February 24, 2003 under the number I-2976.

## Patentansprüche

1. Isoliertes oder gereinigtes Polynukleotid, bestehend aus der Sequenz SEQ ID NO: 1 oder einem Fragment von dieser, welches aus wenigstens 10 aufeinander folgenden Nukleotiden der Sequenz SEQ ID NO: 1 gebildet wird und eine die Transkription fördernde Aktivität aufweist.

2. Isoliertes oder gereinigtes Polynukleotid, bestehend aus der Sequenz SEQ ID NO: 2 oder einem Fragment von dieser, welches aus wenigstens 10 aufeinander folgenden Nukleotiden der Sequenz SEQ ID NO: 2 gebildet wird und eine die Transkription fördernde Aktivität aufweist.

3. Polynukleotid nach Anspruch 1 oder 2, **dadurch gekennzeichnet, dass** es eine die Transkription fördernde Aktivität in Vogelzellen, vorzugsweise in Geflügelzellen und insbesondere in Hühnerzellen aufweist.

4. Rekombinante DNA, welche eines der in den Ansprüchen 1 bis 3 definierten Polynukleotide umfasst.

5. Rekombinante DNA nach Anspruch 4, **dadurch gekennzeichnet, dass** sie außerdem eine zu transkribierende Sequenz umfasst.

6. Rekombinante DNA nach Anspruch 5, **dadurch gekennzeichnet, dass** die zu transkribierende Sequenz eine cDNA eines nicht-umhüllten RNA-Virus ist.

7. Rekombinante DNA nach Anspruch 6, **dadurch gekennzeichnet, dass** die zu transkribierende Sequenz eine cDNA eines RNA-Virus von negativer Polarität ist.

8. Rekombinante DNA nach Anspruch 7, **dadurch gekennzeichnet, dass** das RNA-Virus von negativer Polarität ein Orthomyxovirus oder ein Paramyxovirus ist.

9. Rekombinante DNA nach Anspruch 8, **dadurch gekennzeichnet, dass** das Orthomyxovirus ein Influenza-Virus, vorzugsweise vom Typ A, ist.

10. Rekombinante DNA nach Anspruch 9, **dadurch gekennzeichnet, dass** sie eine vRNA eines Orthomyxovirus, wie eines Grippevirus, und vorzugsweise eine vRNA, welche unter den vRNA-Segmenten 1 bis 8 des Influenzavirus ausgewählt wird, exprimiert.

11. Rekombinante DNA nach Anspruch 8, **dadurch gekennzeichnet, dass** das Paramyxovirus aus der Gattung der Rubulaviren, wie dem Mumpsvirus und dem Newcastle-disease-Virus, der Gattung der Morbilliviren, wie dem Masernvirus, der Gattung Pneumovirus, wie dem Respiratory Syncytial Virus, und der Gattung der Metapneumoviren ausgewählt wird.

12. Vektor, welcher ein Polynukleotid nach einem der Ansprüche 1 bis 3 und/oder eine rekombinante DNA nach einem der Ansprüche 4 bis 11 umfasst.

13. Wirtszelle, welche einen Vektor nach Anspruch 12 umfasst.

14. Wirtszelle nach Anspruch 13, **dadurch gekennzeichnet, dass** sie von Vogel-Ursprung ist.

15. Wirtszelle nach Anspruch 14, **dadurch gekennzeichnet, dass** sie eine Geflügelzelle und insbesondere eine Hühnerzelle ist.

16. Embryoniertes Ei von Vogel-Ursprung, umfassend einen Vektor nach Anspruch 12.

17. Verwendung von wenigstens einem der folgenden Elemente für die Herstellung eines rekombinanten RNA-Virus von negativer Polarität:
- ein Polynukleotid nach einem der Ansprüche 1 bis 3;
- eine rekombinante DNA nach einem der Ansprüche 4 bis 11;
- ein Vektor nach Anspruch 12;
- eine Wirtszelle nach einem der Ansprüche 13 bis 15; und
- ein embryoniertes Ei nach Anspruch 16.

18. Verfahren zur Herstellung von rekombinanten Grippeviren durch inverse Genetik, **dadurch gekennzeichnet, dass** es die folgenden Schritte umfasst:
a) Einführung in eine Vogelzelle oder in ein embryoniertes Ei von Vogel-Herkunft von wenigstens einem Vektor, welcher eine rekombinante DNA, wie in Anspruch 9 definiert, umfasst, und von Vektoren, welche die Proteine PA, PB1, PB2 und NP eines Grippevirus exprimieren;
b) Anwendung von Bedingungen, welche die Expression von rekombinanten Grippeviren erlauben, bei der Zelle oder bei dem Ei, die bzw. das in a) erhalten worden ist; und
c) Gewinnung der in b) erhaltenen rekombinanten Grippeviren.

19. Verfahren nach Anspruch 18, **dadurch gekennzeichnet, dass** die Zelle eine Geflügelzelle und insbesondere eine Hühnerzelle ist.

20. Verfahren nach Anspruch 18, **dadurch gekennzeichnet, dass** das Grippevirus das Influenzavirus, vorzugsweise vom Typ A, ist.

21. Verfahren zur Herstellung von nicht-umhüllten rekombinanten Viren durch inverse Genetik, **dadurch gekennzeichnet, dass** es die folgenden Schritte umfasst:
a) Einführung in eine Vogelzelle oder in ein embryoniertes Ei von Vogel-Herkunft von einem oder mehreren Vektoren, wobei der oder die Vektoren eine rekombinante DNA nach einem der Ansprüche 4 bis 11 umfasst bzw. umfassen und die DNAs die für die Transkription/Replikaton der viralen RNA erforderlichen Proteine exprimieren;
b) Anwendung von Bedingungen, welche die Expression von rekombinanten Viren erlauben, bei der Zelle oder bei dem Ei, die bzw. das in a) erhalten worden ist; und
c) Gewinnung der in b) erhaltenen rekombinanten Viren.

22. Polynukleotid nach Anspruch 1 oder 2, **dadurch gekennzeichnet, dass** es in dem Stamm pGEM-ChPoll-C15-E. coli 1305, der bei der CNCM am 24. Februar 2003 unter der Nummer 1-2976 hinterlegt worden ist, enthalten ist.


### LISTE DE SEQUENCES

<110> INSTITUT PASTEUR
   CENTRE NATIONAL DE LA RECHERCHE SCIENTIFIQUE (CNRS)
   UNIVERSITE DE PARIS 7
<120> PROMOTEUR DE L'ARN POLYMERASE I DE POULET ET SON UTILISATION
<130> D21975
<150> CA 2 421 086
   <151> 2003-02-28
<160> 8
<170> PatentIn version 3.2
<210> 1
   <211> 1261
   <212> ADN
   <213> Gallus gallus
<220>
   <223> Séquence promotrice de l'ARN polymerase I de poulet
<400> 1
   ccggtggtcc cggccccgtc cgactcggtc gcttcgcgga ggtggctgga ggtcgctgcc      60
   gtggcggctg gggcacggcg gaacggtcta accggctccg gcgggccccg tccgcctcgg     120
   tcgctgctcg gcggctgcta ggggtcgctg ccggggtggc tggggcacgg cggaacggtc     180
   tacccgggtc cggcgggcgc cgtccggctc ggtcgctccg cggaggaggc taggggtcgc     240
   tgccggggcg tctcggaaac ggcggaacgg tctacccggc tccggcgggc cccgtccgcc     300
   tcggtcgctc cgcgccggcg gcggctagag gtcgctgccg tgtcggctcg gaaacggcgg     360
   aacggtctac ccggctccga cgggcgccgt ccggctcggt agctccgcgg cggcggctag     420
   aggtcgctgc cgtgtcggct cggaaacggc ggaacggtct acccggctcc ggcgggcgcc     480
   gtccggctcg gtctctccgc ggcggcggct agaggtcgct gccgtgtcgg ctcggaaacg     540
   gcggaacggt ctacccgggt ccgacgggcg ccgtccggct cggtctctcc gcggcggcgg     600
   ctagaggtcg ctgccgtgtc ggctcggaaa cggcggaacg gtctacccgg ctccgacggg     660
   ccccgtccgg ctcggtcgct ccgcggcggc ggctaggggt cgctgccggg gcgtctcgga     720
   aacggcggaa cggtctaccc gggtgctacc gactcgcgct ctccgcggcg gcggctagag     780
   gtcgctgccg gggcggcttg cgatccgcgt ccaggtctac ccggtttcgg attgtcttgg     840
   ccgctctggc tgtggggggg ggcgctacag ctccggagct gccagaggcg tcgctgtaat     900
   tttgtacctc cagttacgtc gaggtaaacc tcggctgccg tcggagccgc tgccggtagt     960
   cggcgcctat ggggctagaa cgtttttttc ggatgcctta tatgttcgtc tgtaggagcg    1020
   agtgaggact cggctccggt agtggcggtg agcgggcgct cgcgagcagg gttgaccggc    1080
   cggccgccta gagaggggat cggcggcggc ggcggcggct ttctcgggca tcggttcgtt    1140
   cgatcggtcc ggtcgcttcg gtttgtccgt cgctcctcat cccgcagctc tgtcctgggc    1200
   taaggcggtt ttgcaggcga gcagcgaaaa aaagccggag aaggcgatca ctagtgcggc    1260
   c                                                                    1261
<210> 2
   <211> 674
   <212> ADN
   <213> Gallus gallus
<220>
   <223> Séquence promotrice de l'ARN polymerase I de poulet
<400> 2
   tccgcggcgg cggctagagg tcgctgccgt gtcggctcgg aaacggcgga acggtctacc     60
   cggctccgac gggccccgtc cggctcggtc gctccgcggc ggcggctagg ggtcgctgcc    120
   ggggcgtctc ggaaacggcg gaacggtcta cccgggtgct accgactcgc gctctccgcg    180
   gcggcggcta gaggtcgctg ccggggcggc ttgcgatccg cgtccaggtc tacccggttt    240
   cggattgtct tggccgctct ggctgtgggg gggggcgcta cagctccgga gctgccagag    300
   gcgtcgctgt aattttgtac ctccagttac gtcgaggtaa acctcggctg ccgtcggagc    360
   cgctgccggt agtcggcgcc tatggggcta gaacgttttt ttcggatgcc ttatatgttc    420
   gtctgtagga gcgagtgagg actcggctcc ggtagtggcg gtgagcgggc gctcgcgagc    480
   agggttgacc ggccggccgc ctagagaggg gatcggcggc ggcggcggcg gctttctcgg    540
   gcatcggttc gttcgatcgg tccggtcgct tcggtttgtc cgtcgctcct catcccgcag    600
   ctctgtcctg ggctaaggcg gttttgcagg cgagcagcga aaaaaagccg gagaaggcga    660
   tcactagtgc ggcc                                                      674
<210> 3
   <211> 26
   <212> ADN
   <213> Séquence artificielle
<220>
   <223> Oligonucléotide dérivé d'ARN ribosomal de poulet
<400> 3
   gagtcagccc tcgacacaag cttttg 26
<210> 4
   <211> 22
   <212> ADN
   <213> Séquence artificielle
<220>
   <223> Oligonucléotide dérivé d'ARN ribosomal de poulet
<400> 4
   ctactggcag gatcaaccag gt 22
<210> 5
   <211> 24
   <212> ADN
   <213> Séquence artificielle
<220>
   <223> Oligonucléotide dérivé d'ARN ribosomal de poulet
<400> 5
   tagaggagaa cgcgacctcg agac 24
<210> 6
   <211> 23
   <212> ADN
   <213> Séquence artificielle
<220>
   <223> Oligonucléotide complémentaire de la séquence CAT transcrite à partir du plasmide pGEM-(AgeI-SacI)-CAT(+)
<400> 6
   atgttcttta cgatgcgatt ggg 23
<210> 7
   <211> 18
   <212> ADN
   <213> Séquence artificielle
<220>
   <223> Amorce dérivée du promoteur de l'ARN polymérase I de poulet
<400> 7
   ggccggtcaa ccctgctc 18
<210> 8
   <211> 38
   <212> ADN
   <213> Séquence artificielle
<220>
   <223> Oligonucléotide dérivé d'un produit de transcription correspondant à un site d'initiation du promoteur de l'ARN polymérase I de poulet
<400> 8
   ttatatgttc gtctgtagga gcgagtgagg actcggct 38